(19) Europäisches Patentamt
European Patent Office
Office européen des brevets

(11) Veröffentlichungsnummer: **0 330 131 A1**

# (12) EUROPÄISCHE PATENTANMELDUNG

(21) Anmeldenummer: **89102944.9**

(22) Anmeldetag: **21.02.89**

(51) Int. Cl.$^4$: **C07D 487/16 , C07D 471/06 , C07D 471/22 , C07D 487/22 , C08K 5/34 , //(C07D487/16, 251:00,209:00),(C07D471/06, 221:00,221:00),(C07D471/22, 251:00,221:00,209:00,209:00), (C07D487/22,339:00,239:00, 209:00,209:00)**

(30) Priorität: **24.02.88 DE 3805758**

(43) Veröffentlichungstag der Anmeldung:
**30.08.89 Patentblatt 89/35**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Aumueller, Alexander, Dr.**
**An der Marlach 5**
**D-6705 Deidesheinm(DE)**
Erfinder: **Neumann, Peter, Dr.**
**Poststrasse 28**
**D-6800 Mannheim 31(DE)**
Erfinder: **Trauth, Hubert**
**Milanstrasse 5**
**D-6724 Dudenhofen(DE)**

## (54) Polycyclische Verbindungen und deren Verwendung.

(57) Die Erfindung betrifft polycyclische Verbindungen der allgemeinen Formel (I)

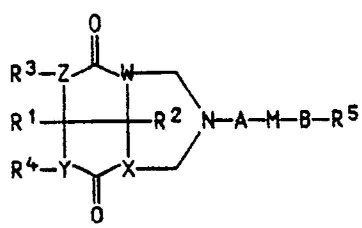

(I),

in der
$R^1$ und $R^2$ unabhängig voneinander für Wasserstoff, Alkyl, Cycloalkyl, gegebenenfalls substituiertes Phenyl, Pyridyl oder Phenylalkyl oder $R^1$ und $R^2$ zusammen für eine Tri- oder Tetramethylengruppe, W, X, Y und Z unabhängig voneinander für C-$R^6$ oder Stickstoff, wobei mindestens ein W, X, Y oder Z C-$R^6$ ist und $R^6$ Wasserstoff, -$CO_2R^7$, -CON$R^7R^8$, Cyan oder Hydroxymethyl und $R^7$ und $R^8$ Wasserstoff, Alkyl, gegebenenfalls substituiertes Phenyl, Cycloalkyl, einen 5- oder 6-gliedrigen Heterocyclus oder Phenylalkyl bedeuten, $R^3$ und $R^4$ für Wasserstoff oder $R^3$ und $R^4$ zusammen für eine Gruppierung der Formel

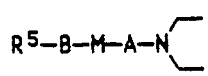

,

EP 0 330 131 A1

A für eine chemische Bindung, Alkylen,

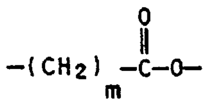, 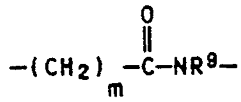, 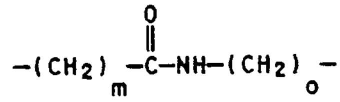

oder Cycloalkylen, worin m und o Zahlen von 1 bis 20 und $R^9$ Alkyl, Cycloalkyl, Phenylalkyl oder gegebenenfalls substituiertes Phenyl bedeuten, M für eine Gruppierung der Formel

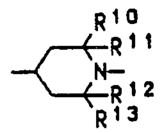

wobei M sowohl mit dem Stickstoffatom als auch mit dem Kohlenstoffatom an A gebunden sein kann und worin $R^{10}$, $R^{11}$, $R^{12}$ und $R^{13}$ unabhängig voneinander Alkyl darstellen oder $R^{10}$ und $R^{11}$ oder $R^{12}$ und $R^{13}$ zusammen eine Tetra- oder Pentamethylengruppe bilden, B für eine chemische Bindung, Alkylen, Phenylalkylen oder für durch Carbonyl, Carbonamid oder Carbonester unterbrochenes Alkylen und $R^5$ für Wasserstoff, Cyan, Hydroxy,

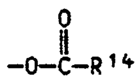, 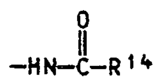, 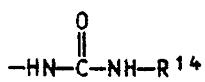, 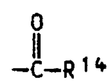, 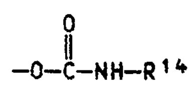,

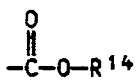 oder 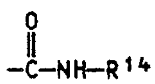 stehen, worin

stehen, worin

$R^{14}$ Wasserstoff, Alkyl, Cycloalkyl, Phenylalkyl, Phenyl oder einen 5- bzw. 6-gliedrigen Heterocyclus bedeutet, oder M-B-$R^5$ für eine Gruppierung der Formeln

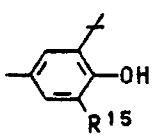, 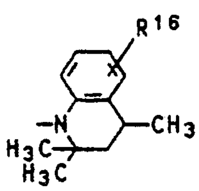, 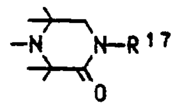, 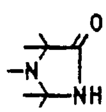 oder 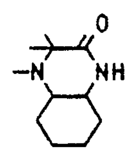

steht, wobei A dann keine chemische Bindung sein darf und $R^{15}$ Alkyl und $R^{16}$ Wasserstoff, Alkyl oder Alkoxy und $R^{17}$ Wasserstoff oder Alkyl bedeuten.

Die Verbindungen (I) besitzen außerordentlich gute stabilisierende Eigenschaften, haben keine Eigenfarbe und sind mit organischen Polymeren gut verträglich. Sie haben außerdem einen niedrigen Dampfdruck und sind stabil gegen thermische Zersetzung.

## Polycyclische Verbindungen und deren Verwendung

Es ist bekannt, daß Polyalkylpiperidinderivate und sterisch gehinderte Phenole organische Polymere von Zerstörung durch Licht und Wärme schützen.

Polycyclische Stabilisatoren sind z.B. aus der EP-A 213570 bekannt.

Unbefriedigend ist bei den polycyclischen Stabilisatoren des Standes der Technik oft deren Verträglichkeit mit Polyolefinen und anderen Kunststoffen, die Dauer der Schutzwirkung, deren Flüchtigkeit und deren thermische Zersetzung beim Einarbeiten in die Polymeren bei erhöhter Temperatur.

Der Erfindung lag die Aufgabe zugrunde, neue Stabilisatoren zur Verfügung zu stellen, welche die vorstehenden Nachteile nicht aufweisen.

Die Aufgabe wird mit den neuen kondensierten Heterocyclen gelöst. Dementsprechend betrifft die Erfindung kondensierte heterocyclische Verbindungen der allgemeinen Formel I

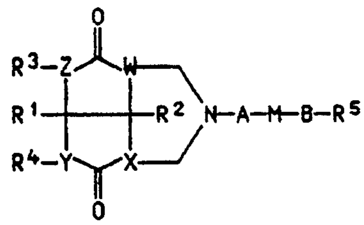 (I),

in der

$R^1$ und $R^2$ unabhängig voneinander für Wasserstoff, $C_1$-$C_{20}$-Alkyl, $C_5$-$C_{12}$-Cycloalkyl, gegebenenfalls substituiertes Phenyl, Pyridyl oder $C_7$-$C_{12}$-Phenylalkyl oder

$R^1$ und $R^2$ zusammen für eine Tri- oder Tetramethylengruppe,

W, X, Y und Z unabhängig voneinander für C-$R^6$ oder Stickstoff, wobei mindestens ein W, X, Y oder Z C-$R^6$ bedeutet und worin

$R^6$ Wasserstoff, $-CO_2R^7$, $-CONR^7R^8$, Cyan oder Hydroxymethyl und

$R^7$ und $R^8$ Wasserstoff, $C_1$-$C_{20}$-Alkyl, gegebenenfalls substituiertes Phenyl, $C_5$-$C_{12}$-Cycloalkyl, einen 5- oder 6-gliedrigen Heterocyclus oder $C_7$-$C_{12}$-Phenylalkyl bedeuten,

$R^3$ und $R^4$ für Wasserstoff oder

$R^3$ und $R^4$ zusammen für eine Gruppierung der Formel

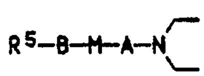 ,

A für eine chemische Bindung, $C_1$-$C_{20}$-Alkylen,

$$-(CH_2)_m-\overset{O}{\overset{\|}{C}}-O-,\quad -(CH_2)_m-\overset{O}{\overset{\|}{C}}-NR^9-,\quad -(CH_2)_m-\overset{O}{\overset{\|}{C}}-NH-(CH_2)_o-$$

oder Cycloalkylen, worin

m und o Zahlen von 1 bis 20 und

$R^9$ $C_1$-$C_{20}$-Alkyl, $C_5$-$C_{12}$-Cycloalkyl, $C_7$-$C_{12}$-Phenylalkyl oder gegebenenfalls substituiertes Phenyl bedeuten,

M für eine Gruppierung der Formel

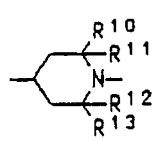

wobei M sowohl mit dem Stickstoffatom als auch mit dem Kohlenstoffatom an A gebunden sein kann und

worin
$R^{10}$ $R^{11}$, $R^{12}$ und $R^{13}$ unabhängig voneinander $C_1$-$C_4$-Alkyl darstellen oder
$R^{10}$ und $R^{11}$ oder $R^{12}$ und $R^{13}$ zusammen eine Tetra- oder Pentamethylengruppe bilden,
B für eine chemische Bindung, $C_1$-$C_{20}$-Alkylen, $C_7$-$C_{18}$-Phenylalkylen oder für durch Carbonyl, Carbonamid oder Carbonester unterbrochenes $C_2$-$C_{20}$-Alkylen und
$R^5$ für Wasserstoff, Cyan, Hydroxy,

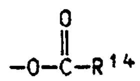, 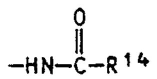, 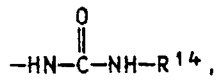, 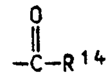, 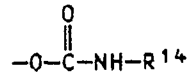,

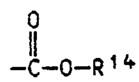 oder 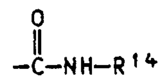 stehen, worin

$R^{14}$ Wasserstoff, $C_1$-$C_{22}$-Alkyl, $C_5$-$C_{12}$-Cycloalkyl, $C_5$-$C_{15}$-Phenylalkyl, Phenyl oder einen 5- bzw. 6-gliedrigen Heterocyclus bedeutet, oder M-B-$R^5$ für eine Gruppierung der Formeln

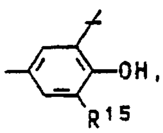, 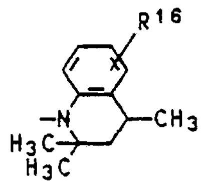, 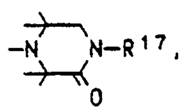, 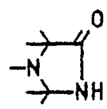 oder 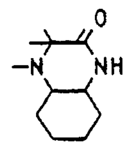

steht, wobei A dann keine chemische Bindung sein kann und
$R^{15}$ $C_1$-$C_4$-Alkyl,
$R^{16}$ Wasserstoff, $C_1$-$C_4$-Alkyl oder $C_1$-$C_4$-Alkoxy und
$R^{17}$ Wasserstoff oder $C_1$-$C_{12}$-Alkyl bedeuten.

Die erfindungsgemäßen Verbindungen besitzen außerordentlich gute stabilisierende Eigenschaften, haben keine Eigenfarbe, sind gut verträglich mit organischen Polymeren, zeigen einen niedrigen Dampfdruck und sind stabil gegen thermische Zersetzung.

Alkylreste für $R^1$, $R^2$ und $R^7$ bis $R^{17}$ können linear oder verzweigt sein.

Im einzelnen sind als Alkyl zu nennen: Methyl, Ethyl, Propyl, iso-Propyl, Butyl, iso-Butyl, Pentyl, Hexyl, Lauryl, Stearyl.

Cycloalkylreste für $R^1$, $R^2$, $R^7$, $R^8$, $R^9$ und $R^{14}$ sind beispielsweise Cyclopentyl, Cyclohexyl, Cycloheptyl, Cyclooctyl und Cyclododecyl.

Phenylalkylreste für $R^1$, $R^2$, $R^7$, $R^8$, $R^9$ und $R^{14}$ sind beispielsweise Benzyl, Methylbenzyl, 2- und 1-Phenylethyl, 1-, 2- und 3-Phenylpropyl und 1-, 2-, 3- und 4-Phenylbutyl.

Als gegebenenfalls substituiertes Phenyl kommen für $R^1$, $R^2$, $R^7$, $R^8$, $R^9$ und $R^{10}$, Phenyl und durch $C_1$-$C_4$-Alkyl, $C_1$-$C_4$-Alkoxy, Chlor, N, N-Di-$C_1$-$C_4$-alkylamino, $C_1$-$C_4$-Alkanoylamino und Phenoxy ein- oder zweifach oder durch Methylendioxy oder Ethylendioxy substituiertes Phenyl in Betracht. Im einzelnen z.B. in Betracht: Phenyl, Tolyl, Ethylphenyl, Methoxyphenyl, Ethoxyphenyl, Chlorphenyl, Dimethoxyphenyl, Bromphenyl, Acetamidophenyl, N,N-Dimethylaminophenyl, Phenoxyphenyl, N,N-Diethylaminophenyl und 3,4-Methylendioxyphenyl.

Als heterocyclischer Rest ist für $R^7$ oder $R^8$ z.B. der 2,2,6,6-Tetramethylpiperidinyl-(4)-rest zu nennen.

Für $R^1$ und $R^2$ sind Wasserstoff, Methyl, Ethyl und Phenyl bevorzugt. Weiterhin sind Verbindungen der allgemeinen Formel (I) bevorzugt, in denen $R^1$ und $R^2$ zusammen eine Tetramethylengruppe bilden.

$R^6$ kann beispielsweise sein:

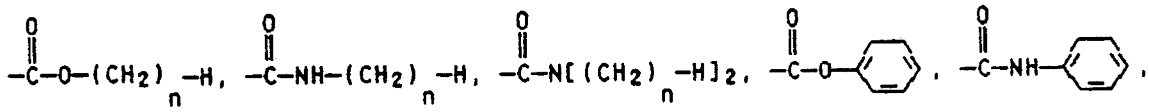

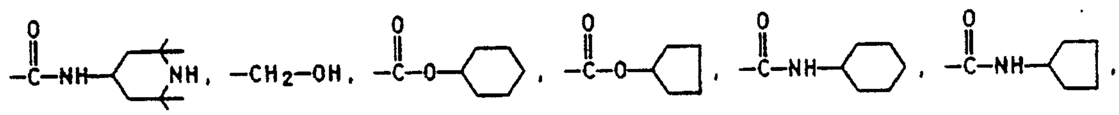

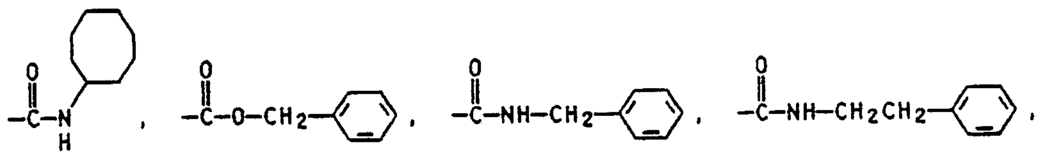

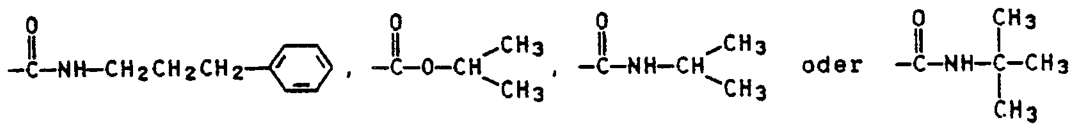

oder

mit n = 0 bis 20.

Vorzugsweise steht $R^6$ für Wasserstoff, Cyan, $-CO-OCH_3$, $-CO-OCH_2CH_3$ oder $-CO-NH_2$.

Brückenglieder A und B sind beispielweise:

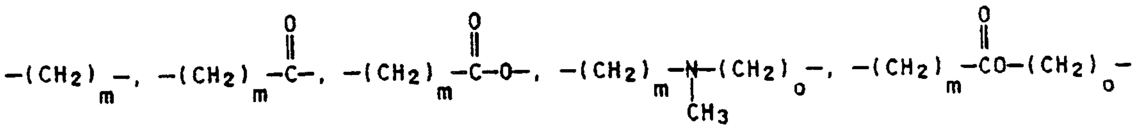

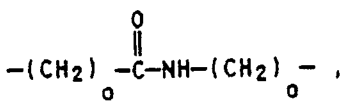

worin m und o für Zahlen von 1 bis 20 stehen. Bevorzugt sind für A und B die chemische Bindung $-CH_2-$ und $-CH_2-CH_2-$.

$R^5$ kann beispielsweise sein:

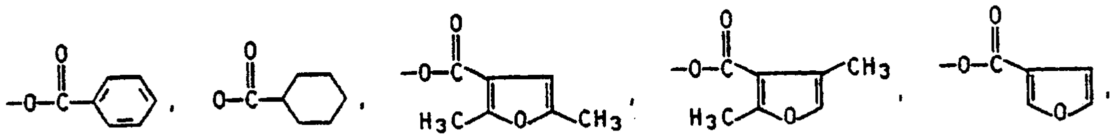

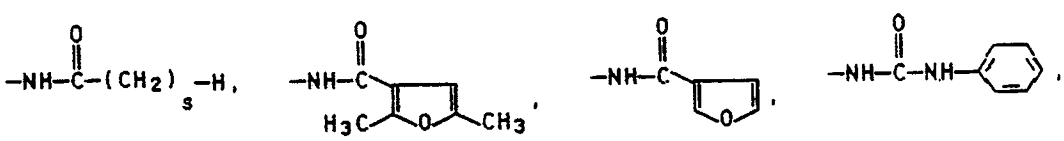

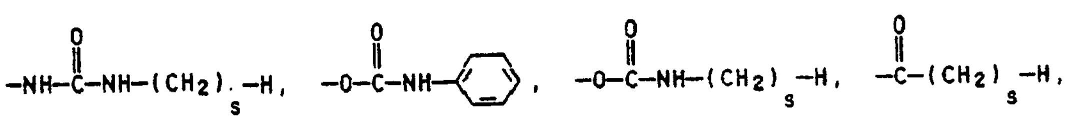

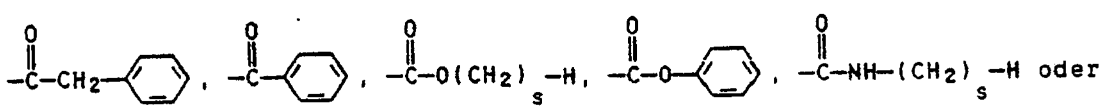

oder

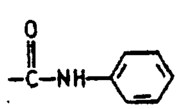

mit s = 1 bis 20.

$R^5$ steht vorzugsweise für $C_2$- bis $C_{18}$-Alkanoyloxy oder $C_2$- bis $C_{18}$-Alkanoylamino wie -O-CO-$CH_3$, -O-CO-$CH_2CH_3$, -O-CO-$(CH_2)_2$-$CH_3$, -O-CO-$(CH_2)_3$-$CH_3$, $^3O^3CO^3(CH'')§^3CH§$, $^3O^3CO^3(CH'')_5$-$CH_3$, -O-CO-$(C_2)_6$-$CH_3$,-O-CO-$(CH_2)_7$-$CH_3$, -O-CO-$(CH_2)_8$-$CH_3$, -O-CO-$(CH_2)_9$-$CH_3$, -O-CO-$(CH_2)_{10}$-$CH_3$, -O-CO-$(CH_2)_{11}$-$CH_3$, -O-CO-$(CH_2)_{12}$-$CH_3$, -O-CO-$(CH_2)_9$-$CH_3$, -O-CO-$(CH_2)_{14}$-$CH_3$, -O-CO-$(CH_2)_{15}$ -$CH_3$, -O-CO-$(CH_2)_{16}$-$CH_3$, -NH-CO-$CH_3$, -NH-CO-$CH_2CH_3$, -NH-CO-$(CH_2)_2$-$CH_3$, -NH-CO-$(CH_2)_3$-$CH_3$, -NH-CO-$(CH_2)_4$-$CH_3$, $^3NH^3CO^3$-$(CH'')_5$-$CH_3$, -NH-CO-$(CH_2)_6$-$CH_3$, -NH-CO-$(CH_2)_7$-$CH_3$, -NH-CO-$(CH_2)_8$-$CH_3$, -NH-CO-$(CH_2)_9$-$CH_3$, -NH-CO-$(CH_2)_{10}$-$CH_3$, -NH-CO-$(CH_2)_{11}$ -$CH_3$, -NH-CO-$(CH_2)_{12}$-$CH_3$, -NH-CO-$(CH_2)_{13}$-$CH_3$, -NH-CO-$(CH_2)_{14}$-$CH_3$, -NH-CO-$(CH_2)_{15}$-$CH_3$, -NH-CO-$(CH_2)_{16}$-$CH_3$ oder

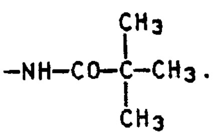

Besonders bevorzugt sind für $R^5$ Wasserstoff, Hydroxy und Cyan.

Die Gruppierung M-B-$R^5$ kann auch z.B. für

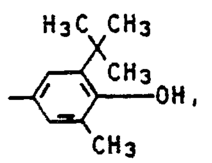,
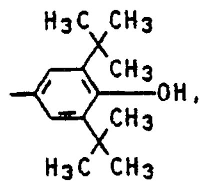,
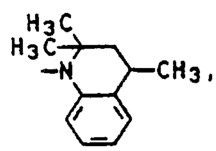,
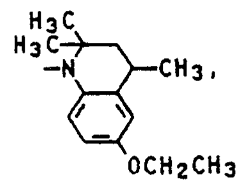,
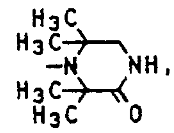,

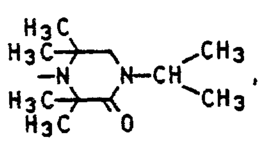,
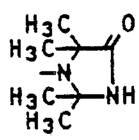
oder
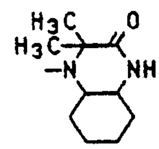

stehen, wobei A dann keine chemische Bindung sein kann.

Die Verbindungen der allgemeinen Formel (I) lassen sich beispielsweise durch Reaktion von Formaldehyd oder Paraformaldehyd und Bicyclen der allgemeinen Formel (II) mit Aminen der allgemeinen Formel (III) oder (IV) herstellen.

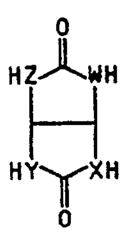
(II)

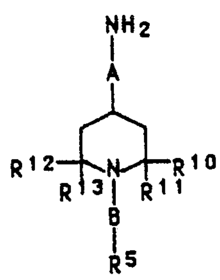
(III)

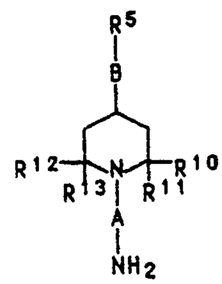
(IV)

In den Formeln haben X, Y, Z, W, $R^5$, $R^{10}$ bis $R^{13}$, A und B die oben angegebenen Bedeutungen.

Dabei arbeitet man vorzugsweise in einem Lösungsmittel bei Temperaturen zwischen 40 und 120 °C, insbesondere zwischen 60 und 100 °C.

Bevorzugte Lösungsmittel sind Alkohole wie Methanol, Ethanol, Propanol, iso-Propanol, Butanol oder iso-Butanol und Wasser. Setzt man Gemische von verschiedenen Aminen der allgemeinen Formeln III und/oder IV ein, so erhält man Gemische von Verbindungen der allgemeinen Formel (I). Die beiden Gruppierungen A-M-B-$R^5$, die in einem Molekül enthalten sein können, können dann gleich oder verschieden sein.

Verbindungen der allgemeinen Formel (I), die noch reaktive Gruppen, wie z.B. Hydroxy, Amino, Cyan oder Ester, enthalten, können durch an sich literaturbekannte Verfahren wie Acylierung, Alkylierung, Reduktion, Umsetzen mit Isocyanaten, Umestern oder Umsetzen mit Aminen in andere Verbindungen der allgemeinen Formel (I) umgewandelt werden.

Die Ausgangsmaterialien der allgemeinen Formel (II) sind teilweise bekannt (DE-A-2 300 638), teilweise neu. Die Herstellung der neuen Verbindungen (II) ist in den Beispielen beschrieben.

Die erfindungsgemäßen Verbindungen können in Form der freien Basen, als Hydrate oder als Salze vorliegen. Geeignete Anionen stammen z. B. von anorganischen Säuren und insbesondere organischen Carbonsäuren sowie organischen Sulfonsäuren.

Anorganische Anionen sind z.B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanoat, Cyclohexanoat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen (I) stabilisieren organisches Material, speziell Kunststoffe, gegen den Abbau durch Licht und Wärme. Sie sind auch wirksam als Metalldesaktivator. Den zu stabilisierenden Kunststoffen wird (I) in einer Konzentration von 0,01 bis 5 Gew.-% vorzugsweise von 0,02 bis 2 Gew.-% vor, während oder nach der Polymerbildung zugesetzt.

Die durch die Verbindungen (I) stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel sowie Pigmente und Füllstoffe.

Die neuen Verbindungen (I) können nach bekannten Methoden und in den bekannten Vorrichtungen in die zu stabilisierenden Kunststoffe, gegebenenfalls mit weiteren Stabilisierungsmitteln und/oder anderen Zusätzen eingearbeitet werden.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel sind z.B. zu nennen: 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butyl-phenyl)-butan, 1,3,5-Trimethyl-2,4,6-tris-(3′,5′-di-tert.-butyl-4′-hydroxy-benzyl)-benzol, 1,3,5-Tris-(3′,5′-di-tert.-butyl-4-hydroxybenzyl)-isocyanurat, 1,3,5-Tris-[β-(3′,5′-di-tert.-butyl-4′-hydroxyphenyl)-propionyloxy-ethyl]-isocyanurat, 1,3,5-Tris-(2′,6′,di-methyl-3′-hydroxy-4′-tert.-butylbenzyl)-isocyanurat und Pentaerythrit-tetrakis-[β-(3,5-di-tert.-butyl-4-hydroxy-phenyl)-propionat].

Als phosphorhaltige Antioxidantien kommen z.B. in Betracht: Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4′-biphenylendiphosphit.

Als Schwefel enthaltende Antioxidationsmittel sind z.B. zu nennen: Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat).

Weitere Antioxidantien und Lichtstabilisatoren, die zusammen mit den erfindungsgemäßen Verbindungen (I) verwendet werden können, sind z. B. 2-(2′-Hydroxyphenyl)-benztriazole, 2-Hydroxybenzophenone, Arylester von Hydroxybenzoesäuren, α-Cyanozimtsäurederivate, Nickelverbindungen, Oxalsäuredianilide oder Benzimidazolcarbonsäureanilide.

Als organische Polymere, die mit den erfindungsgemäßen Verbindungen stabilisiert werden können, sind z.B. zu nennen:

Polymere von Mono- und Diolefinen, wie z. B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polysytrol;

Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methylmethacrylat;

ABS-, MBS- und ähnliche Polymere;

Halogenhaltige Polymere, wie z. B. Polyvinylchlorid, Polyvinylfluorid, Polyvinylidenfluorid sowie deren

Copolymere;
Polymere die sich von α•β-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen bzw. deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weiterhin können Überzüge, die mit Hilfe von Lacken erzeugt werden, mit den Verbindungen (I) gegen den Abbau durch Licht und Wärme stabilisiert werden. Von diesen sind Einbrennlackierungen, insbesondere Fahrzeuglackierungen, vorzugsweise Zweischichtlackierungen, besonders hervorzuheben. Auch hier können zusätzlich die bereits genannten Antioxidantien und Lichtschutzmittel verwendet werden.

Die erfindungsgemäßen Verbindungen können in den Lacken in fester oder gelöster Form zugesetzt werden. Ihre gute Löslichkeit in Lacksystemen ist dabei von besonderem Vorteil.

Besonders gute stabilisierende Eigenschaften in Verbindung mit hervorragender Verträglichkeit zeigen die erfindungsgemäßen Verbindungen (I) in Polyolefinen, insbesondere in Ethylen- und Propylenpolymerisaten, in Polyurethanen und Lacken.

Die Erfindung soll durch die folgenden Beispiele zusätzlich weiter erläutert werden.

## Herstellbeispiele

### Beispiel 1

11,6 g 2,8-Diazabicyclo-[3,3,0]octan-3,7-dion (DE-A-2 300 638), 5 g Paraformaldehyd und 12,9 g 2,2,6,6-Tetramethyl-piperidin-4-amin wurden in 80 ml Wasser zusammengegeben und 2 Stunden auf 80 °C erhitzt. Das Lösungsmittel wurde im Vakuum abdestilliert, der Rückstand aus Aceton umkristallisiert. Man erhielt 14,0 g der Verbindung der Formel

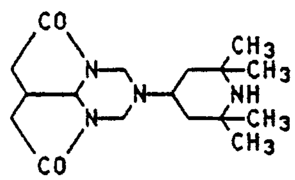

als farblosen Feststoff vom Schmp. 187 °C.

### Beispiel 2

18,5 g 3,7-Dihydroxybicyclo-[3,3,0]octa-2,6-dien-2,4,6,8-tetracarbonsäuremethylester (Org. Synth. 64, 27 [1985]) und 6 g Paraformaldehyd wurden 0,5 Stunden in 150 ml Methanol gekocht. Anschließend wurden 15,5 g 2,2,6,6-Tetramethylpiperidin-4-amin in 150 ml Methanol zugetropft und eine weitere Stunde am Rückfluß gekocht. Man goß auf Wasser und saugte den ausgefallenen Niederschlag ab. Man isolierte 4,2 g der Verbindung der Formel

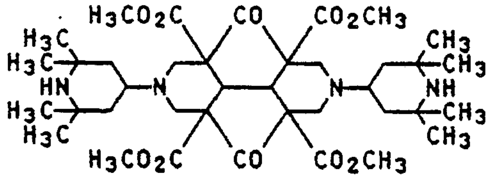

als farblosen Feststoff vom Schmp. 120 - 122 °C.

Durch Zugabe von Katalysatoren, z.B. Lewatit® S 100, Piperidin oder Pyridin konnte die Ausbeute auf ca. das Doppelte gesteigert werden.

Beispiel 3

20 g 1,5-Dimethyl-3,7-dioxo-2,8-diaza-cis-bicyclo-[3,3,0]octan-4,6-dicarbonitril (Chem. Ber. 108, 3256 [1975]), 11 g Paraformaldehyd und 28,5 g 2,2,6,6-Tetramethylpiperidin-4-amin wurden in 250 ml Wasser 4 Stunden auf 80 °C erhitzt. Der ausgefallene Niederschlag wurde heiß abgesaugt, mit heißem Wasser gewaschen und getrocknet. Man isolierte 41 g der Verbindung der Formel

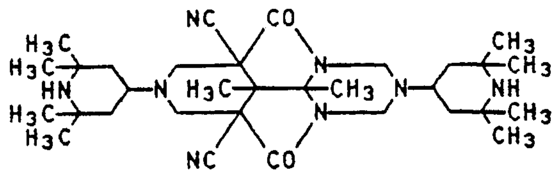

als farblosen Feststoff vom Schmp. 276 °C (Zers.).

Die Verbindung enthielt 0,5 Mol Kristallwasser.

Beispiel 4

21,8 g 1,5-Dimethyl-3,7-dioxo-2,6-diaza-cis-bicyclo-[3,3,0]octan-4,8-dicarbonitril (Chem. Ber. 108, 3247 [1975]), 12 g Paraformaldehyd und 31,2 g 2,2,6,6-Tetramethylpiperidin-4-amin wurden in 300 ml iso-Butanol 4 Stunden am Wasserabscheider gekocht. Nach dem Abkühlen wurde abgesaugt, mit iso-Butanol und Petrolether gewaschen und getrocknet. Man isolierte 50 g der Verbindung der Formel

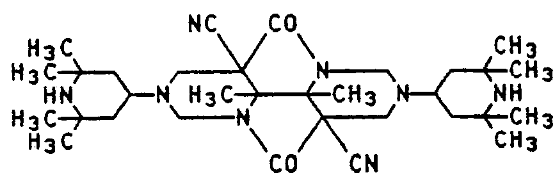

als farblosen Feststoff vom Schmp. 284 °C (Zers.).

Die Substanz enthielt 0,5 Mol Kristallwasser.

Beispiel 5

51,3 g eines 1:1-Gemisches aus 3,7-Dioxo-1,5-diphenyl-2,6-diaza-cis-bicyclo-[3,3,0]octan-4,8-dicarbonitril und 3,7-Dioxo-1,5-diphenyl-2,8-diaza-cis-bicyclo-[3,3,0]octan-4,6-dicarbonitril (Chem. Ber. 108, 3262 [1975]), 18 g Paraformaldehyd und 46,8 g 2,2,6,6-Tetramethylpiperidin-4-amin wurden in 600 ml iso-Butanol 3,5 Stunden am Wasserabscheider gekocht. Der Niederschlag wurde bei 70 °C abgesaugt und mit iso-Butanol gewaschen. Man isolierte 64 g eines 1:1-Gemisches der Verbindungen der Formeln

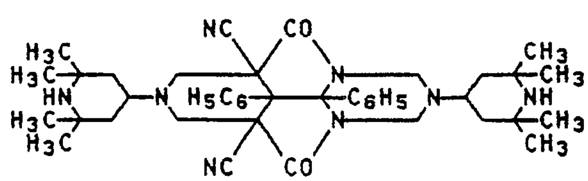

und

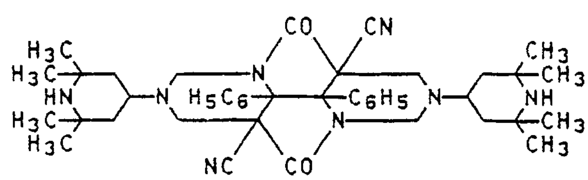

vom Schmp. 278 °C (Zers.).

Die Substanz enthielt 0,5 Mol Kristallwasser.

Beispiel 6

Zu 91,2 g 5-Hydroxy-4,5-dimethyl-2-oxo-3-pyrrolin-3-carbonitril (Chem. Ber. 108, 3262 [1975]) und 108 g einer 30 %igen methanolischen Natriummethanolatlösung wurde bei 0 °C eine Lösung von 59,4 g Cyanessigsäuremethylester in 150 ml Methanol getropft. Man ließ 18 Stunden bei 0 bis 5 °C stehen, gab 52 ml konzentrierte Salzsäure zu, rührte 15 Minuten nach und saugte ab. Der Rückstand wurde in 300 ml Wasser zum Sieden erhitzt, abgekühlt, abgesaugt, mit Wasser nachgewaschen und getrocknet. Man isolierte 33,2 g der Verbindung der Formel

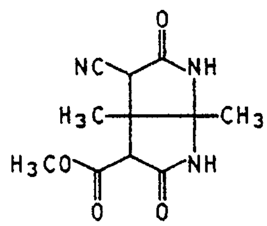

vom Schmp. 262 °C (Zers.).

Beispiel 7

Zu 12,55 g des Produktes aus Beispiel 6 und 6 g Paraformaldehyd in 100 ml Methanol wurden 15,6 g 2,2,6,6-Tetramethyl-piperidin-4-amin getropft. Dabei erhielt man eine Lösung, aus der nach 2,5 Stunden Kochen am Rückfluß das Produkt ausfiel. Der Niederschlag wurde abgesaugt, mit 100 ml Methanol gewaschen und getrocknet. Man isolierte 17,7 g der Verbindung der Formel

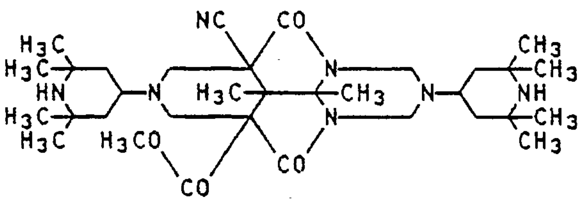

als farblosen Feststoff vom Schmp. 277 °C (Zers.).

Die Substanz enthielt 0,5 Mol Kristallwasser.

Beispiel 8

11,4 g des Produkts aus Beispiel 6 wurden mit 70 ml 2,2,6,6-Tetramethyl-piperidin-4-amin 3 Stunden auf 100 °C, anschließend 2,5 Stunden auf 130 °C erhitzt. Nach Zugabe von weiteren 50 ml 2,2,6,6-Tetramethyl-piperidin-4-amin wurde noch 1 Stunde auf 130 °C gehalten. Nach dem Abkühlen wurde mit 500 ml Petrolether versetzt, der ausgefallene Niederschlag abgesaugt, mit Petrolether gewaschen und getrocknet. Man isolierte 14 g der Verbindung der Formel

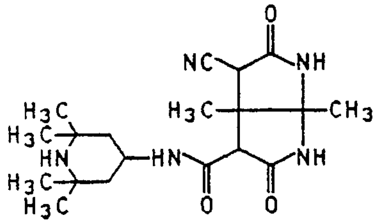

vom Schmp. 262 bis 263 °C (Zers.).

Beispiel 9

10 g des Produkts aus Beispiel 8 wurden mit 3,12 g Paraformaldehyd und 8,1 g 2,2,6,6-Tetramethyl-piperidin-4-amin in 100 ml Wasser 7 Stunden auf 80 °C gehalten. Der Niederschlag wurde bei Raumtemperatur abgesaugt, mit Wasser gewaschen und aus Acetonitril umkristallisiert. Man isolierte 4,5 g der Verbindung der Formel

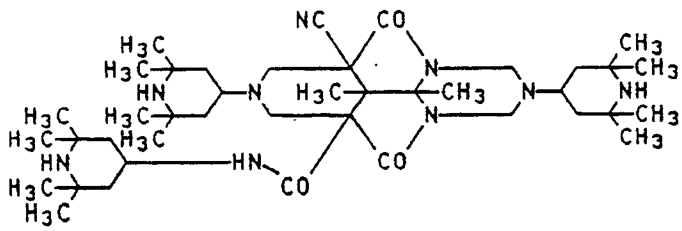

vom Schmp. 291 °C (Zers.).

Beispiel 10

Zu 36 g Harnstoff und 24 ml konzentrierter Salzsäure in 500 ml Methanol wurden 91,2 g 5-Hydroxy-4,5-dimethyl-2-oxo-3-pyrrolin-3-carbonitril (Chem. Ber, 108, 3262 [1975]) gegeben. Man erhitzte 12 Stunden auf 50 °C und engte anschließend ein. Der ölige Rückstand wurde mit 500 ml Aceton ausgekocht, heiß abgesaugt, mit 150 ml Methanol und 500 ml Aceton verrührt, abgesaugt, mit Aceton gewaschen und getrocknet. Man isolierte 34,8 g der Verbindung der Formel

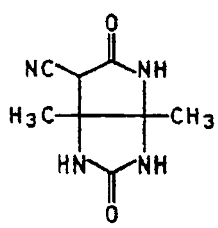

vom Schmp. 274 bis 275 °C (Zers.).

Beispiel 11

5,8 g des Produkts aus Beispiel 10, 3,6 g Paraformaldehyd und 9,3 g 2,2,6,6-Tetramethyl-piperidin-4-amin wurden in Wasser 2 Stunden auf 80 °C erhitzt. Der ausgefallene Niederschlag wurde abgesaugt, mit Wasser ausgekocht, abgesaugt, mit Wasser gewaschen und getrocknet. Man isolierte 11,3 g der Verbindung der Formel

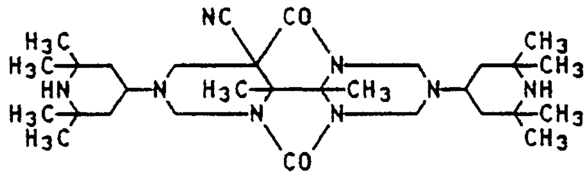

vom Schmp. 277 °C (Zers.).

Beispiel 12

a) 27,6 g 5-Hydroxy-2-oxo-4,5-diphenyl-3-pyrrolin-3-carbonitril (Chem. Ber. 108, 3262 [1975]) und 36 g Harnstoff wurden mit 1 g p-Toluolsulfonsäure in 250 ml Toluol 30 Stunden am Wasserabscheider gekocht. Nach dem Abkühlen wurde abgesaugt, mit Wasser und iso-Propanol ausgekocht, abgesaugt, mit Aceton gewaschen und getrocknet. Man isolierte 20,6 g der Verbindung der Formel

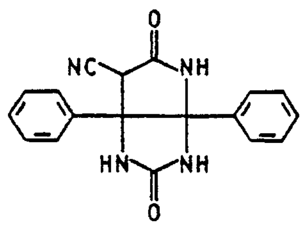

vom Schmp. 297 °C (Zers.).

b) 41,4 g 5-Hydroxy-2-oxo-4,5-diphenyl-3-pyrrolin-3-carbonitril und 180 g Harnstoff wurden gemischt und auf 150 °C erhitzt. Nach 0,75 Stunden ließ man abkühlen und tropfte bei 130 bis 100 °C 250 ml Wasser zu. Bei 70 °C wurde abgesaugt und mit Wasser gewaschen. Man isolierte 25 g eines Produkts, das mit dem aus Beispiel a) identisch war.

Beispiel 13

10 g des Produkts aus Beispiel 11 wurden mit 3,75 g Paraformaldehyd und 9,8 g 2,2,6,6-Tetramethyl-piperidin-4-amin in 300 ml Wasser 5 Stunden auf 80 °C erhitzt. Die Reaktionsmischung wurde heiß abgesaugt, der Rückstand aus DMF umkristallisiert. Nach dem Trocknen isolierte man 14 g der Verbindung der Formel

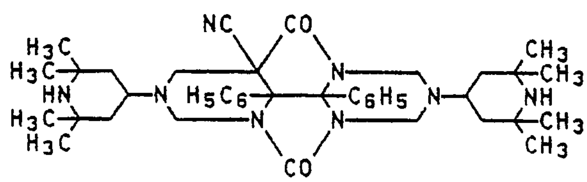

vom Schmp. 300 °C.

Beispiel 14

134,4 g Cyclohexandion-1,2, 100,8 g Cyanacetamid und 3 ml Piperidin wurden in 1350 ml Dichlormethan 9 Stunden am Wasserauskreiser gekocht. Die Reaktionsmischung wurde abgesaugt, der Rückstand mit Petrolether gewaschen und getrocknet. Man isolierte 153 g der Verbindung der Formel

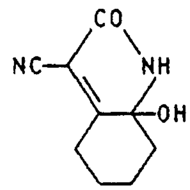

vom Schmp. 169 °C (Zers.).

Beispiel 15

Zu 35,6 g des Produkts aus Beispiel 14 und 36 g einer 30 %igen methanolischen Natriummethanolatlösung in 200 ml Methanol wurden bei 0 °C 13,2 g Malondinitril in 50 ml Methanol getropft. Die Mischung wurde 3 Stunden bei 0 bis 5 °C gerührt, anschließend wurden 20 ml konzentrierte Salzsäure zugetropft. Der ausgefallene Niederschlag wurde abgesaugt, mit Petrolether gewaschen, mit Wasser ausgerührt, abgesaugt, mit Wasser gewaschen und getrocknet. Man isolierte 34 g der Verbindung der Formel

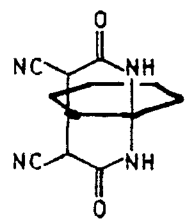

vom Schmp. 289 °C (Zers.).

Beispiel 16

10,1 g des Produkts aus Beispiel 15, 5 g Paraformaldehyd und 13,1 g 2,2,6,6-Tetramethyl-piperidin-4-amin wurden in 150 ml Wasser 2 Stunden auf 80 °C erhitzt. Nach dem Abkühlen wurde der ausgefallene Niederschlag abgesaugt, mit Wasser gewaschen und aus Acetonitril umkristallisiert. Man isolierte 15 g der Verbindung der Formel

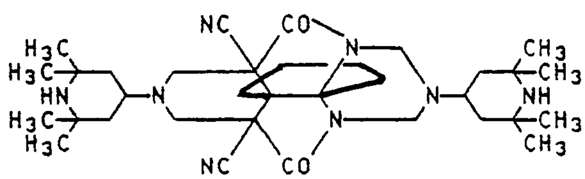

vom Schmp. 279 °C (Zers.)

Beispiel 17

Zu 26,7 g des Produkts aus Beispiel 14 und 27 g einer 30 %igen methanolischen Natriummethanoloatlösung tropfte man bei 0 °C 14,9 g Cyanessigsäuremethylester in 40 ml Methanol. Die Mischung wurde 3 Stunden bei 0 bis 5 °C und 16 Stunden bei Raumtemperatur gerührt. Unter Eiskühlung wurden 16 ml konzentrierte Salzsäure zugetropft, der ausgefallene Niederschlag wurde abgesaugt, mit Methanol gewaschen, mit Wasser verrührt, abgesaugt, mit Wasser gewaschen und getrocknet. Man isolierte 16 g der Verbindung der Formel

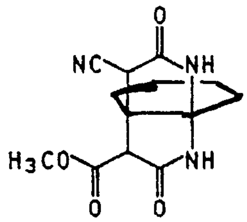

vom Schmp. 262 °C (Zers.).

Beispiel 18

12,3 g des Produkts aus Beispiel 17, 5,3 g Paraformaldehyd und 13,7 g 2,2,6,6-Tetramethyl-piperidin-4-amin wurden in 150 ml Methanol 4 Stunden am Rückfluß gekocht. Nach dem Abkühlen wurde der ausgefallene Niederschlag abgesaugt, mit Methanol gewaschen und aus Acetonitril umkristallisiert. Man isolierte 21 g der Verbindung der Formel

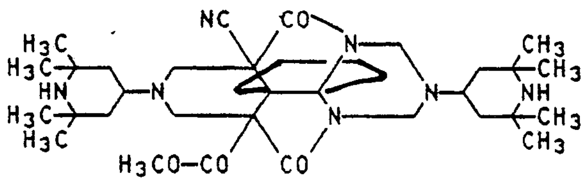

vom Schmp. 252 °C (Zers.)

Beispiel 19

7,73 g des Produktes aus Beispiel 6, 3,7 g Paraformaldehyd und 9,6 g 2,2,6,6-Tetramethyl-piperidin-4-amin wurden 3,5 Stunden auf 80 °C erhitzt. Nach dem Abkühlen wurde abgesaugt, der Rückstand mit 200 ml Wasser ausgekocht und abgesaugt. Man isolierte 6,0 g der Verbindung der Formel

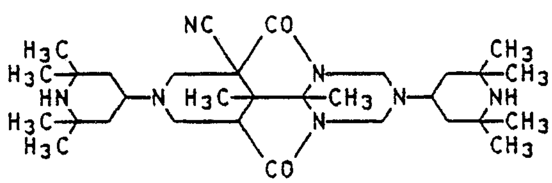

vom Schmp. 299 °C.

Beispiel 20

120 g Pentandion-2,3 und 50,4 g Cyanacetamid wurden unter Zusatz von 1 ml Piperidin in 600 ml Dichlormethan am Wasserabscheider 7 Stunden gekocht. Der ausgefallene Niederschlag wurde abgesaugt und mit Methylenchlorid gewaschen. Man isolierte 57 g eines Isomerengemisches der Verbindungen der Formeln

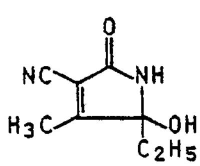

und

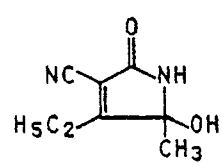

im Verhältnis 9:1 ($^{13}C$-NMR). Der Schmelzpunkt betrug 157 °C (Zers.).

Beispiel 21

Zu einer Lösung von 17 g des Produktes aus Beispiel 20 und 18,4 g 30 %iger methanolischer Natriummethanolatlösung in 100 ml Methanol wurde bei 0 °C eine Lösung von 6,75 g Malondinitril in 40 ml Methanol getropft. Man rührte 3 Stunden bei 0 bis 5 °C und 1 Stunde bei Raumtemperatur. Mit konzentrierter Salzsäure wurde angesäuert, der ausgefallene Niederschlag abgesaugt, mit Wasser gewaschen und bei 80 °C getrocknet. Man erhielt 15,0 g der Verbindung der Formel

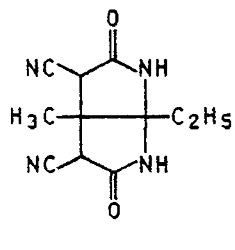

vom Schmp. 266 °C (Zers.).

Beispiel 22

12,0 g des Produkts aus Beispiel 21, 20,6 g 30 %ige wäßrige Formaldehydlösung und 16,1 g 2,2,6,6-Tetramethylpiperidin-4-amin wurden in 150 ml Wasser 4,5 Stunden auf 80 °C erhitzt. Der ausgefallene Niederschlag wurde heiß abgesaugt, mit Wasser gewaschen und aus Acetonitril umkristallisiert. Nach dem Trocknen isolierte man 11,0 g der Verbindung der Formel

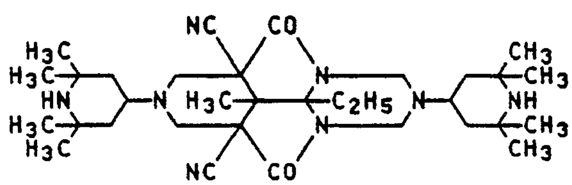

vom Schmp. 292 °C (Zers.).

Beispiel 23

10,0 g 1,5-Dimethyl-3,7-dioxo-2,8-diaza-bicyclo-[3.3.0]octan-4-carbonitril (Chem. Ber. 108, 3262 [1975]), 3,1 g Paraformaldehyd und 8,1 g 2,2,6,6-Tetramethylpiperidin-4-amin wurden in 100 ml Methanol 12 Stunden gekocht. Nach Zugabe von weiteren 6,2 g Paraformaldehyd und 16,2 g 2,2,6,6-Tetramethylpiperidin-4-amin wurde eine weitere Stunde gekocht. Die flüchtigen Bestandteile wurden im Wasserstrahlvakuum entfernt, der Rückstand in Toluol heiß gelöst und die Lösung in 1 l n-Hexan eingetropft. Der langsam kristallisierende Niederschlag wurde bei -20 °C abgesaugt, mit n-Heptan ausgekocht und getrocknet. Man isolierte 13,3 g der Verbindung der Formel

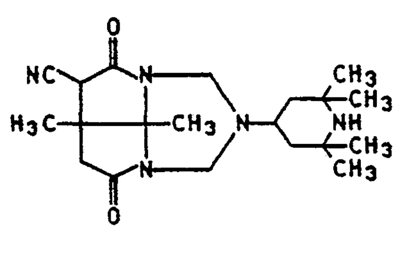

vom Schmp. 172 bis 173 °C.

Beispiel 24

9,0 g 5-Methyl-3,7-dioxo-1-phenyl-2,8-diaza-cis-bicyclo-[3.3.0]-octan-4,6-dicarbonitril (Chem. Ber. 108, 3262 [1975]), 12,8 g 30 %ige wäßrige Formaldehydlösung und 10,0 g 2,2,6,6-Tetramethylpiperidin-4-amin wurden in 125 ml Wasser 3,5 Stunden auf 80 °C erhitzt. Nach dem Abkühlen wurde der ausgefallene Niederschlag abgesaugt und aus Methanol umkristallisiert. Man isolierte 10,0 g der Verbindung der Formel

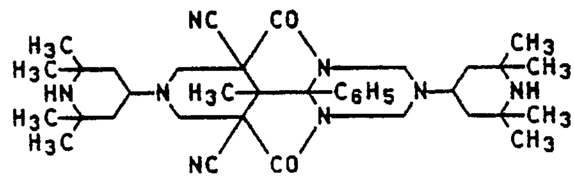

vom Schmp. 295 °C (Zers.). Die Verbindung enthielt 0,5 Mol Kristallwasser.

Beispiel 25

8,0 g 5-Methyl-3,7-dioxo-1-phenyl-2,8-diaza-cis-bicyclo-[3.3.0]octan-4,6-dicarbonitril (Chem. Ber. 108, 3262 [1975]), 11,2 g 30 %ige wäßrige Formaldehydlösung und 17,5 g 1-β-Aminoethyl-2,2,6,6-tetramethylpiperidin-4-ol (DE-OS 32 08 570) wurden in 125 ml Wasser 5 Stunden auf 80 °C erhitzt. Nach dem Abkühlen wurde der ausgefallene Niederschlag abgesaugt, mit Wasser gewaschen und aus n-Butanol umkristallisiert. Man erhielt 6,3 g der Verbindung der Formel

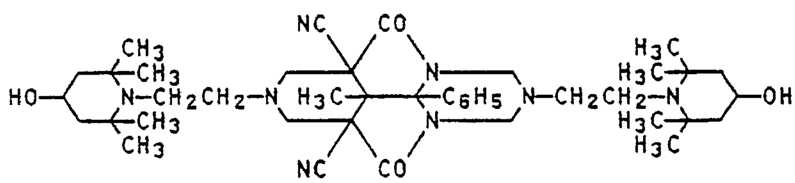

vom Schmp. 239 °C. Die Verbindung enthielt 0,5 Mol Kristallwasser.

Beispiel 26

16,6 g des Produkts aus Beispiel 20 wurden mit 6,6 g Harnstoff und 4 ml konzentrierter Salzsäure in 100 ml Methanol 4 Stunden und nach Zugabe von weiteren 7 g Harnstoff 16 Stunden gekocht. Die flüchtigen Bestandteile wurden im Wasserstrahlvakuum entfernt, der Rückstand mit Wasser angerieben und abgesaugt. Nach zweimaligem Auskochen mit Aceton erhielt man 4,8 g der Verbindung der Formel

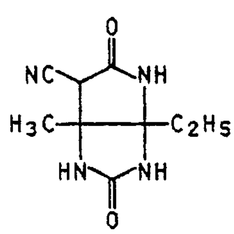

Beispiel 27

4,8 g des Produkts aus Beispiel 26 wurden mit 9,2 g 30 %iger wäßriger Formaldehydlösung und 7,2 g 2,2,6,6-Tetramethylpiperidin-4-amin in 100 ml Wasser 4 Stunden auf 80 °C erhitzt. Nach dem Abkühlen wurde abgesaugt, mit Wasser gewaschen und aus Acetonitril und aus Methanol umkristallisiert. Man erhielt 1,7 g der Verbindung der Formel

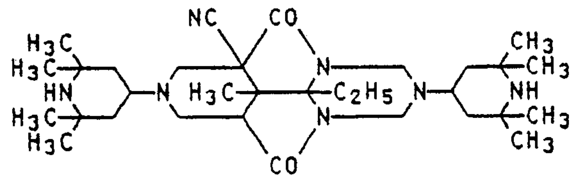

vom Schmp. 284 °C. Die Verbindung enthielt 0,5 Mol Kristallwasser.

Beispiel 28

51,3 g eines 1:1-Gemisches aus 3,7-Dioxo-1,5-diphenyl-2,6-diaza-cis-bicyclo[3.3.0]octan-4,8-dicarbonitril und 3,7-Dioxo-1,5-diphenyl2,8-diaza-cis-bicyclo-[3.3.0]octan-4,8-dicarbonitril (Chem. Ber. 108, 3262 [1975]), 16,0 g 30 %ige wäßrige Formaldehydlösung und 16,1 g 1-$\beta$-Aminoethyl-2,2,6,6-tetramethylpiperidin-4-ol (DE-OS 3208570) wurden in 125 ml Wasser 2,5 Stunden auf 80 °C erhitzt. Nach dem Abkühlen wurde abgesaugt, mit Wasser gewaschen und der Rückstand aus Methanol umkristallisiert. Man erhielt 15,0 g eines 1:1-Gemisches der Verbindungen der Formeln

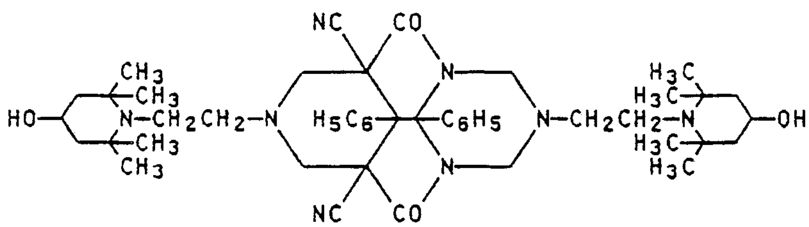

und

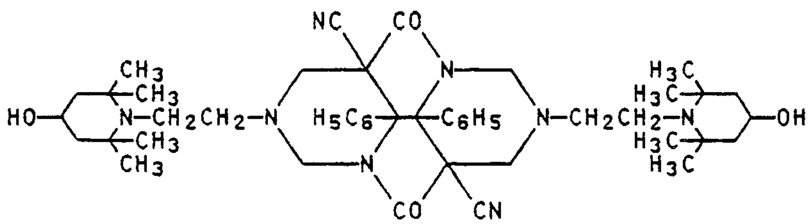

vom Schmp. 247 °C. Die Verbindung enthielt 1 Mol Kristallwasser.

Beispiel 29

27 g des Produkts aus Beispiel 12, 34 g 30 %ige wäßrige Formaldehydlösung und 36 g 1-$\beta$-Aminoethyl-2,2,6,6-tetramethylpiperidin-4-ol (DE-OS 3208570) wurden in 300 ml Wasser und 30 ml Methanol 2 Stunden auf 80 °C erhitzt. Nach Zugabe von 100 ml Methanol wurde weitere 2,5 Stunden auf 80 °C gehalten. Nach dem Abkühlen wurde abgesaugt, mit Wasser gewaschen und der Rückstand aus Methanol umkristallisiert. Man isolierte 23 g der Verbindung der Formel

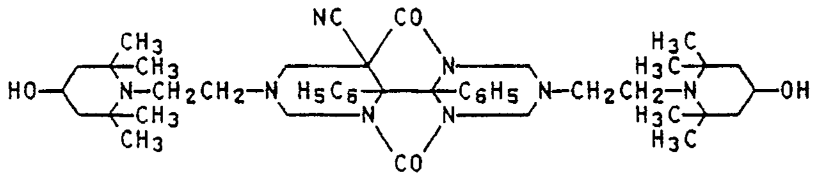

vom Schmp. 247 °C (Zers.). Die Verbindung enthielt 0,5 Mol Kristallwasser.

Beispiel 30

7,9 g des Produkts aus Beispiel 10, 16,4 g 30 %ige wäßrige Formaldehydlösung und 17,5 g 1-β-Aminoethyl-2,2,6,6-tetramethylpiperidin-4-ol (DE-OS 3208570) wurden in 125 ml Wasser 1,5 Stunden auf 80 °C erhitzt. Anschließend wurde abgekühlt, abgesaugt, mit Wasser gewaschen und aus n-Butanol umkristallisiert. Man isolierte 10,0 g der Verbindung der Formel

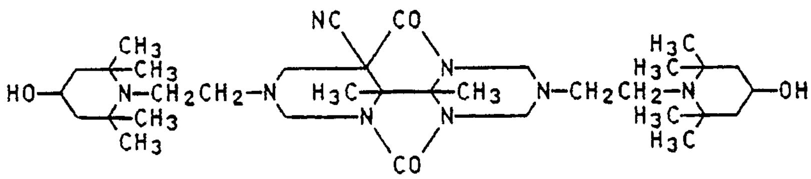

vom Schmp. 280 °C (Zers.).

Beispiel 31

10,0 g des Produkts aus Beispiel 6, 4,8 g Paraformaldehyd und 16,1 g 1-β-Aminoethyl-2,2,6,6-tetramethylpiperidin-4-ol (DE-OS 3208570) wurden in 140 ml Methanol 2,75 Stunden gekocht. Nach dem Abkühlen wurde abgesaugt, mit Methanol gewaschen und aus Acetonitril umkristallisiert. Nach dem Trocknen erhielt man 13,0 g der Verbindung der Formel

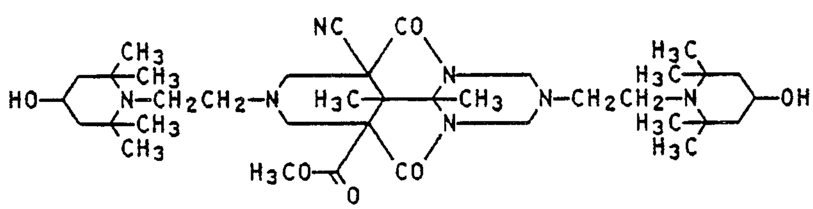

vom Schmp. 254 °C (Zers.).

Beispiel 32

9,6 g des Produkts aus Beispiel 15, 16,0 g 30 %ige wäßrige Formaldehydlösung und 17,5 g 1-β-Aminoethyl-2,2,6,6-tetramethyl-piperidin-4-ol wurden in 125 ml Wasser und 15 ml Methanol 5 Stunden auf 80 °C erhitzt. Nach dem Abkühlen wurde abgesaugt, mit Wasser gewaschen und mit Methanol ausgekocht. Man isolierte 1,8 g der Verbindung der Formel

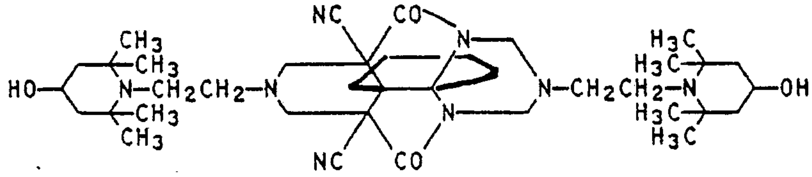

vom Schmp. 271 °C (Zers.). Die Verbindung enthielt 0,5 Mol Kristallwasser.

Beispiel 33

21 g des Produkts aus Beispiel 30 wurden bei 10 bis 20 °C in 325 ml Acetylchlorid eingetragen. Nach 6 Stunden Rühren bei Raumtemperatur wurde mit 250 ml Petrolether verdünnt und abgesaugt. Der Niederschlag wurde nach dem Trocknen in 400 ml Wasser gelöst, die wäßrige Lösung filtriert und mit 50 %iger Natronlauge unter Kühlung alkalisch gestellt. Der ausgefallene Niederschlag wurde abgesaugt, mit Wasser gewaschen und bei 90 °C im Wasserstrahlvakuum getrocknet. Man isolierte 16 g der Verbindung der Formel

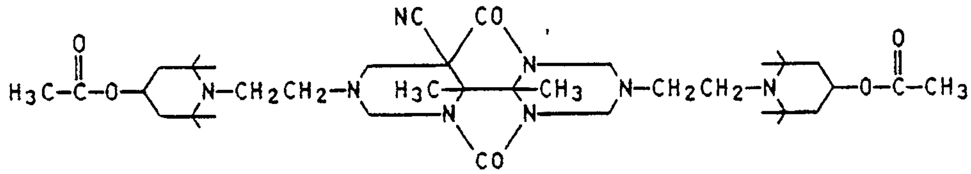

vom Schmp. 242 °C (Zers.).

**Ansprüche**

1. Polycyclische Verbindungen der allgemeinen Formel (I)

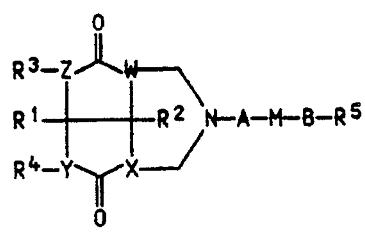

(I),

in der
$R^1$ und $R^2$ unabhängig voneinander für Wasserstoff, $C_1$-$C_{20}$-Alkyl, $C_5$-$C_{12}$-Cycloalkyl, gegebenenfalls substituiertes Phenyl, Pyridyl oder $C_7$-$C_{12}$-Phenylalkyl oder
$R^1$ und $R^2$ zusammen für eine Tri- oder Tetramethylengruppe,
W, X, Y und Z unabhängig voneinander für C-$R^6$ oder Stickstoff, wobei mindestens ein W, X, Y oder Z C-$R^6$ ist und
$R^6$ Wasserstoff, -$CO_2R^7$, -$CONR^7R^8$, Cyan oder Hydroxymethyl und
$R^7$ und $R^8$ Wasserstoff, $C_1$-$C_{20}$-Alkyl, gegebenenfalls substituiertes Phenyl, $C_5$-$C_{12}$-Cycloalkyl, einen 5- oder 6-gliedrigen Heterocyclus oder $C_7$-$C_{12}$-Phenylalkyl bedeuten,
$R^3$ und $R^4$ für Wasserstoff oder
$R^3$ und $R^4$ zusammen für eine Gruppierung der Formel

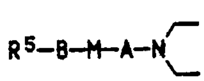

,

A für eine chemische Bindung, $C_1$-$C_{20}$-Alkylen,

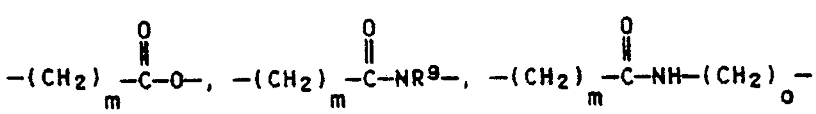

oder Cycloalkylen, worin

m und o Zahlen von 1 bis 20 und
$R^9$ $C_1$-$C_{20}$-Alkyl, $C_5$-$C_{12}$-Cycloalkyl, $C_7$-$C_{12}$-Phenylalkyl oder gegebenenfalls substituiertes Phenyl bedeuten,
M für eine Gruppierung der Formel

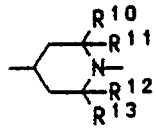

wobei M sowohl mit dem Stickstoffatom als auch mit dem Kohlenstoffatom an A gebunden sein kann und worin
$R^{10}$, $R^{11}$, $R^{12}$ und $R^{13}$ unabhängig voneinander $C_1$-$C_4$-Alkyl darstellen oder
$R^{10}$ und $R^{11}$ oder $R^{12}$ und $R^{13}$ zusammen eine Tetra- oder Pentamethylengruppe bilden,
B für eine chemische Bindung, $C_1$-$C_{20}$-Alkylen, $C_7$-$C_{18}$-Phenylalkylen oder für durch Carbonyl, Carbonamid oder Carbonester unterbrochenes $C_2$-$C_{20}$-Alkylen und
$R^5$ für Wasserstoff, Cyan, Hydroxy,

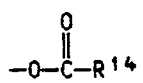, 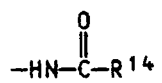, 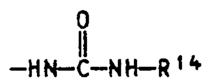, 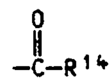, 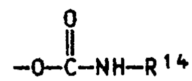,

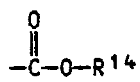 oder 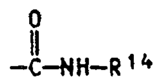 stehen, worin

$R^{14}$ Wasserstoff, $C_1$-$C_{22}$-Alkyl, $C_5$-$C_{12}$-Cycloalkyl, $C_5$-$C_{15}$-Phenylalkyl, Phenyl oder einen 5- bzw. 6-gliedrigen Heterocyclus bedeutet, oder M-B-$R^5$ für eine Gruppierung der Formeln

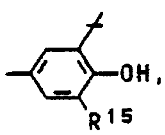, 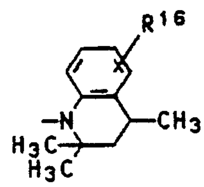, 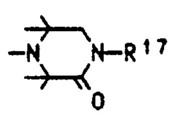, 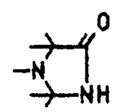 oder 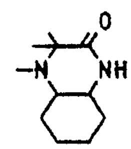

steht, wobei A dann keine chemische Bindung sein darf und
$R^{15}$ $C_1$-$C_4$-Alkyl,
$R^{16}$ Wasserstoff, $C_1$-$C_4$-Alkyl oder $C_1$-$C_4$-Alkoxy und
$R^{17}$ Wasserstoff oder $C_1$-$C_{12}$-Alkyl bedeuten.

2. Verbindungen gemäß Anspruch 1, dadurch gekennzeichnet, daß $R^{10}$, $R^{11}$, $R^{12}$ und $R^{13}$ Methyl bedeuten.

3. Verbindungen gemäß Anspruch 1 oder 2, dadurch gekennzeichnet, daß A und B für eine chemische Bindung, Methylen oder Ethylen stehen.

4. Verbindungen gemäß Anspruch 1, 2 oder 3, dadurch gekennzeichnet, daß die Phenylreste $R^1$, $R^2$, $R^7$, $R^8$ und $R^9$ gegebenenfalls durch $C_1$-$C_4$-Alkyl, $C_1$-$C_4$-Alkoxy, Chlor, N,N-Di-$C_1$-$C_4$-alkylamino, $C_1$-$C_4$-Alkanoylamino oder Phenoxy ein- oder zweifach oder durch Methylendioxy oder Ethylendioxy substituiert sind.

5. Verbindungen gemäß Anspruch 1, 2, 3 oder 4, dadurch gekennzeichnet, daß $R^1$ und $R^2$ für Wasserstoff, Methyl, Ethyl oder Phenyl stehen oder $R^1$ und $R^2$ zusammen eine Tetramethylengruppe bilden.

6. Verbindungen gemäß Anspruch 1, 2, 3, 4 oder 5, dadurch gekennzeichnet, daß $R^6$ für Wasserstoff, Cyan, Carbomethoxy, Carboethoxy, Carbamoyl oder N-4(2,2,6,6-Tetramethylpiperidinyl)-carbamoyl steht.

7. Verbindungen gemäß Anspruch 1, 2, 3, 4, 5 oder 6, dadurch gekennzeichnet, daß $R^5$ für Wasserstoff, Hydroxy, Cyan, $C_2$-$C_{18}$-Alkanoyloxy oder $C_2$-$C_{18}$-Alkanoylamino stehen.

8. Verwendung von Verbindungen gemäß den Ansprüchen 1 bis 7 zum Stabilisieren von organischem Material, insbesondere von Kunststoffen.

9. Verwendung von Verbindungen gemäß den Ansprüchen 1 bis 7 zum Stabilisieren von Polyurethanen, Polyolefinen und Lacken.

10. Verwendung von Verbindungen gemäß den Ansprüchen 1 bis 3 als Metallionendesaktivator.

11. Stabilisiertes organisches Material, enthaltend Verbindungen gemäß den Ansprüchen 1 bis 7.

Europäisches Patentamt

# EUROPÄISCHER RECHERCHENBERICHT

Nummer der Anmeldung

EP 89 10 2944

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP-A-0 272 591 (BASF AG)<br>* Ansprüche 1,4-8 *<br>--- | 1,8-11 | C 07 D 487/16<br>C 07 D 471/06<br>C 07 D 471/22<br>C 07 D 487/22<br>C 08 K 5/34 //<br>(C 07 D 487/16<br>C 07 D 251:00<br>C 07 D 209:00 )<br>(C 07 D 471/06<br>C 07 D 221:00<br>C 07 D 221:00 )<br>(C 07 D 471/22<br>C 07 D 251:00<br>C 07 D 221:00<br>C 07 D 209:00<br>C 07 D 209:00 )<br>(C 07 D 487/22<br>-/- |
| P,A | EP-A-0 272 589 (BASF AG)<br>* Ansprüche 1,10-14 *<br>--- | 1,8-11 | |
| D,A | EP-A-0 213 570 (BASF AG)<br>* Ansprüche 1,3,9,11; Seite 5, Zeile 38 - Seite 6, Zeile 30 *<br>--- | 1,8,9,<br>11 | |
| A | FR-A-2 291 203 (NOVEL HOECHST CHIMIE)<br>* Anspruch 1 *<br>--- | 1 | |
| A | JOURNAL OF ORGANIC CHEMISTRY<br>Band 50, 1985, Seiten 60-62; W.L. MOCK et al.:"A Novel Hexacyclic Ring System from Glycoluril"<br>--- | 1 | |
| D,A | DE-A-2 300 638 (LABORATORIOS MADE S.A.)<br>* Seite 3, Mitte, Formel V *<br>--- | 1 | **RECHERCHIERTE SACHGEBIETE (Int. Cl.4)** |
| D,A | ORGANIC SYNTHESES<br>Band 64, 1985, Seiten 27-38; S.H. BERTZ et al.:"Condensation of dimethyl 1,3-acetone-dicarboxylate with 1,2-dicarbonyl compounds: cis-bicyclo (3.3.0) octane -3,7-diones" * Seite 27, Formelschema *<br>----- | 1 | C 07 D 471/00<br>C 07 D 487/00<br>C 08 K 5/00 |

Der vorliegende Recherchenbericht wurde für alle Patentansprüche erstellt

| Recherchenort | Abschlußdatum der Recherche | Prüfer |
|---|---|---|
| BERLIN | 23-05-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 10 2944

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | | | C 07 D 239:00<br>C 07 D 239:00<br>C 07 D 209:00<br>C 07 D 209:00 )<br>(C 07 D 487/22<br>C 07 D 251:00<br>C 07 D 239:00<br>C 07 D 235:00<br>C 07 D 209:00 ) |
| | | | RECHERCHIERTE SACHGEBIETE (Int. Cl.4) |
| | | | |

Der vorliegende Recherchenbericht wurde für alle Patentansprüche erstellt

| Recherchenort | Abschlußdatum der Recherche | Prüfer |
|---|---|---|
| BERLIN | 23-05-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)